# EUROPEAN PATENT SPECIFICATION

(11) **EP 0 989 859 B1**
(45) Date of publication and mention of the grant of the patent: **13.08.2003**
(21) Application number: 98926325.6
(22) Date of filing: 10.06.1998
(51) Int. Cl.: A61K 38/18, A61K 48/00, C12N 15/85, C12N 5/10, A01K 67/027, A61K 9/00

(54) **CNS NEUROREGENERATIVE COMPOSITIONS AND METHODS OF USE**
ZNS NEUROREGENERATIVE ZUSAMMENSETZUNGEN UND DEREN VERWENDUNGEN
COMPOSITIONS NEUROREGENERATIVES DU SYSTEME NERVEUX CENTRAL ET PROCEDES D'UTILISATION

(30) Priority: 11.06.1997 US 873267
(43) Date of publication of application: 05.04.2000
(73) Proprietor: Acorda Therapeutics, Hawthorne, NY 10531 (US)
(72) Inventor: LOGAN, Ann, Stourport-on-Seven, Worc. D61 39TA (GB); BERRY, Martin, UMDS of Guy's Hospital, London SE1 9RT (GB)
(74) Representative: Hallybone, Huw George
(86) International application number: US9811619
(87) International publication number: WO98056404

(56) References cited:
- WO-A-90/06757
- WO-A-93/08828
- WO-A-94/03199
- WO-A-94/12201
- US-A- 5 602 309
- PATENT ABSTRACTS OF JAPAN vol. 16, no. 172 (C-933), 24 April 1992 & JP 04 018031 A (HIROSHI SAITO), 22 January 1992
- NAKAHARA ET AL.: "Grafts of fibroblasts genetically modified to secrete NGF, BDNF, NT-3 or basic FGF elicit differential responses in the adult spinal cord" CELL TRANSPLANTATION, vol. 5, no. 2, March 1996, pages 191-204, XP002078570

## Description

### BACKGROUND OF THE INVENTION

### Field of the Invention

This invention relates generally to the modulation of neural growth in the central nervous system, and more particularly to compositions, and to their associated methods, for improving CNS neural growth. Specifically, the invention relates to the use of neurotrophic factors, and more to compositions and other formulations containing combinations of neurotrophins to foster and improve such neural growth.

### Description of the Related Art

Despite efforts spanning many years, neural regeneration in the central nervous system (CNS) has never been successfully therapeutically achieved and maintained. Possible explanations for this failure include the following: either (1) trophic molecules essential for regrowth are absent from the CNS or (2) inhibitory molecules are present in CNS neutrophils which arrest axonal growth soon after injury, explaining the initial abortive growth response of damaged axons, or (3) because both phenomena are operating. Recent work supports the first hypothesis by demonstrating that neurotrophins delivered to the cell bodies of injured axons promote the regeneration of their distantly injured axons through the putative inhibitory environment of a CNS myelinated tract. Thus, up to 10% of retinal ganglion cells (RGC) regenerate axons at least 2mm beyond a crush injury into the distal segment of the optic nerve 20 days after implanting a teased segment of sciatic nerve into the vitreous body of the eye (Berry et al., 1996). Acellular peripheral nerve implants have a minimal effect, indicating that growth factors secreted by Schwann cells probably promote the regenerative response of the optic nerve. No scar tissue is deposited in the lesions traversed by regenerating fibers.

Many central and peripheral neurons depend on the target-derived neurotrophins NGF, BDNF and NT-3 for their survival, for axonal growth both *de novo* and after injury, and for the maintenance of transmitter production, acting through the high affinity tyrosine kinase (trk) receptors; trk A, trk B and trk C, respectively, which are expressed in receptive neurons. A low affinity p75 NGF receptor (LNGFR) is widely expressed throughout the CNS and the peripheral nervous system (PNS), but its role in regeneration remains obscure. In both mammals and birds the expression of LNGFR, trk A, trk B, and trk C has been reported in retinal ganglion cells during development. All are down-regulated postnatally; trk B faster than trk C, with trk A and trk B becoming sparsely distributed in large neurons, probably retinal ganglion cells, in the inner nuclear layer (Rodriguez-Tebar et al., 1993; Takahashi et al., 1993; Jelsma et al., 1993; Elkabes et al., 1995; Koide et al., 1995; Richma and Brecha, 1995; Perez and Caminos, 1995). The expression of full length trk B and trk C precedes that of the truncated form of the receptor (Escandon et al., 1994). In culture, BDNF and NT3 have been shown to control the proliferation, differentiation and survival of fetal retinal ganglion cells (Allendoerfer et al., 1994; Castillo et al., 1994; Delarosa et al., 1994). CNTF has also been reported to promote the survival and neurite outgrowth of retinal ganglion cells in culture (Carri et al., 1994). Astrocytes and Müller cells express the mRNA for NGF, BDNF and NT3 at all ages *in vivo* (Elkabes et al., 1995) and, *in vitro,* have trk A and LNGFR and respond to NGF by proliferation (Ikeda & Puro, 1994). Another source of neurotrophins is the target tissue to which the retinal ganglion cells project. For example, BDNF mRNA is expressed in the chick tectum two days before the arrival of retinal ganglion cell axons (Herzog et al., 1994).

Acidic and basic fibroblast growth factors (FGF1 and FGF2) and their high affinity tyrosine kinase receptors FGFR1 and FGFR2 (with equal affinity for both FGFs) are expressed in neurons and glia throughout the CNS and PNS subserving functions including axonal growth, neuronal and glial differentiation and survival, and glial mitogenesis (Baird, 1994). FGF and FGFR are essential for the proliferation and survival of rods and cones during retinal development (e.g., Perry et al., 1995; Lillien, 1994; Tcheng et al., 1994a,b; Ishigooka et al., 1993; Malecaze et al., 1993; Bugra et al., 1993; Mascarelli et al., 1991). In culture, retinal neural proliferation in general is controlled by FGF1 and FGF2 (Lillien and Cepko, 1992), and FGFR2 is localized in retinal ganglion cell axons in the fibre layer of the retina (Torriglia and Blanquet, 1994; Torriglia et al., 1994). Under the influence of FGF1 and FGF2, retinal pigment cells can regenerate the entire neural retina over a critical period of eye development (for review see Park & Hollenberg, 1993).

There has been little work reporting the changes in expression of FGFR and trks in retinal ganglion cells after optic nerve section. Nonetheless, it is known that after optic nerve lesions, BDNF and, to a lesser extent NT3 and CNTF prevent post-traumatic axon die-back in neonates, although no effect is seen when NGF, FGF1 and FGF2 are given alone (Weibel et al., 1995). Intravitreal injection of BDNF and CNTF supports retinal ganglion cells (RGC) survival in adult rats after optic nerve transection (Mey and Thanos, 1993; Mansourrobaey et al., 1994) and, *in vitro,* the neurites of retinal ganglion cells extend for longer distances in the presence of BDNF-transfected fibroblasts compared with the control parent cell line (Takahashi et al., 1993). The ability of neurons to extend neurites is of prime importance in establishing neuronal connections during development. It is also required during regeneration to re-establish connections destroyed as a result of a lesion.

Neurites elongate profusely during development both in the central and peripheral nervous systems of all animal species (Cajal (1928) Degeneration and regeneration in nervous system, Oxford University Press, London). This phenomenon pertains to axons and dendrites. However, in adults, axonal and dendritic regrowth in the central nervous system is increasingly lost with evolutionary progression.

In the peripheral nervous system, after infliction of a lesion, axons of all vertebrate species are able to regrow (Cajal (1928); Martini (1994)). However, in the central nervous system of mammals, neurite regrowth following damage is limited to neuritic sprouting. Regrowth of neuronal processes is, however, possible in lower vertebrate species (Stuermer et al. (1992). In contrast, in the central nervous system, most, if not all, neurons of both higher and lower vertebrate adults possess the potential for neurite regrowth (Aguayo (1985)).

Glial cells are the decisive determinants for controlling axon regrowth. Mammalian glial cells are generally permissive for neurite outgrowth in the central nervous system during development (Silver et al. (1982); Miller et al. (1985); Pollerberg et al. (1985); and in the adult peripheral nervous system (Fawcett et al. (1990). Thus, upon infliction of a lesion, glial cells of the adult mammalian peripheral nervous system can revert to some extent to their earlier neurite outgrowth-promoting potential, allowing them to foster regeneration (Kalderon, 1988; Kliot et al.; Carlstedt et al., 1989). Glial cells of the central nervous system of some lower vertebrates remain permissive for neurite regrowth in adulthood (Stuermer et al., 1992). In contrast, glial cells of the central nervous system of adult mammals are not conducive to neurite regrowth following lesions.

Neurotrophic factors are present during normal development of the nervous system. During such development, neuronal target structures produce limited amounts of specific neurotrophic factors necessary for both the survival and differentiation of the neurons projecting into the structures. The same factors have been found to be involved in the survival and/or maintenance of mature neurons.

However, long term experiments demonstrate that peripheral nerve implants do not maintain regeneration beyond 30 days post lesion (dpl) and by 100 dpl most axons degenerate, presumably because Schwann cells in the peripheral nerve implants stop producing neurotrophic factors at about 20 days post-implantation, possibly due to a lack of axonal contact.

There thus exists a need for regenerative therapies which can promote neural growth so as to enable the damaged or disease nerve to again function.

### SUMMARY OF THE INVENTION

In accordance with the present invention, a composition and corresponding methods are disclosed for the modulation of neural growth and particularly, such growth as can be promoted in the compartment of the central nervous system (CNS), and specifically, in myelinated nerve tissue.

Accordingly, in a first aspect of the present invention, a composition is disclosed for the promotion of neural regeneration in the CNS which composition comprises the combination of two or more neurotrophic agents, embodied in a variety of vehicles, all for the administration to the site in the CNS where regeneration is needed. More particularly, the composition includes the preparation of a quantity of cells such as fibroblasts, that have been transfected to express the multiple neurotrophins when located at the CNS site in question. The composition also extends to pharmaceutical formulations including the neurotrophins of the invention, that may be administered in a variety of known ways, to achieve the regenerative effects in object.

A neurotrophic factor is defined as a substance capable of increasing and/or maintaining survival of a neuron population, and affecting outgrowth of neurites (neuron processes) and certain other metabolic activities of a neuron. Neurotrophic factors are generally described as soluble molecules synthesized in the peripheral targets of neurons and transported to their cell bodies, where they exert their effects. Respresentative neurotropic factors are the neurotrophins selected from the group consisting of nerve growth factor (NGF), acidic fibroblast growth factor (FGF-1), basic fibroblast growth factor (FGF-2), neurotrophin-3 (NT-3), brain-derived neurotrophic factor (BNDF), active fragments thereof, cognates thereof, congeners thereof, mimics, analogs, secreting cells and soluble molecules thereof in an amount sufficient to promote said neural growth for the treatment of injured or diseased central nervous system tissue in a mammal.

Accordingly, it is a principal object of the present invention to provide a composition and method of promoting neural growth that comprises and uses a combination of at least two neurotrophic factors.

A further object of the invention is to provide methods of delivering the neurotrophins of the combination to the patient under treatment.

A still further object of the invention is to provide a method for enhancing neuronal outgrowth of CNS neurons, which includes the secretion of neural adhesion molecule by implanted cells.

Other objects and advantages will become apparent to those skilled in the art from a review of the ensuing detailed description taken with reference to the following illustrative drawings.

### BRIEF DESCRIPTION OF THE DRAWINGS

The file of this patent contains at least one drawing executed in color. Copies of this patent with color drawings will be provided by the Patent and Trademark Office upon request and payment of the necessary fee.
FIGURE 1 is a color photomicrograph showing that in the control groups most RGCs become degenerate in the retina (mean counts of HRP-positive RGC in the two groups = 0-191) and few if any GAP 43 (growth associated protein) positive axons were present in the proximal nerve segment.
FIGURE 2 is a color photomicrograph showing that in the control groups no fibers crossed the lesion site and dense scar material was deposited in the wound.
FIGURE 3 is a color photomicrograph showing that the animals receiving all three Nts show the greatest number of HRP-positive RGCs (25-2762, mean = 603), with significant numbers of GAP 43 positive axons regenerating 3-5 mm into the distal nerve segment.
FIGURE 4 is a color photomicrograph showing that in the animals receiving all three Nts a glia/mesenchymal scar was not formed at the wound site.

### DETAILED DESCRIPTION OF THE PREFERRED EMBODIMENTS OF THE INVENTION

The present invention provides results which reveal several new features of the regenerative response which appear to be common to the CNS as a whole: (1) Regeneration is possible through the putative inhibitory environment of CNS myelinated tracts after delivery of trophic factors to the perikarya of injured axons without a prerequisite for neutralization of an inhibitory substrate. (2) Neurotrophins may promote regeneration by both mobilizing growth cone mechanisms and down-regulating receptors for the inhibitory ligands on growth cones which mediate their collapse (Johnston, 1993; Berry et al., 1994; Keynes & Cook, 1995). (3) Application of trophic molecules via either transfected cells or Schwann cells to the site of injury may sequester regenerating axons and also alter the properties of CNS glia, both of which could impede regrowth over long distances. (4) Regenerative success after peri-neuronal neurotrophin administration is correlated with the suppression of glial/collagen scarring in the lesion which might be explained if: (a) rapid regrowth of axons through the wound is effected by peri-somatic neurotrophin application. (The delay in mobilizing growth, after local application of neurotrophins into the wound, engendered by uptake and retrograde transport to distant somata may thus be crucial for scar deposition, if the early immigration of scar promoting cells into the lesion is impeded by the presence of growing axons); (b) neurotrophins induce the secretion of metalloproteinases and plasminogen activators from growth cones which down-regulate, in the reactive glia and hematogenous cells, the production of transforming growth factor (TGF), a cytokine with a major role in initiating the scarring cascade (Romanic & Madri, 1994; Monard, 1988; Logan et al., 1992).

Thus, the present invention utilizes cells, and particularly fibroblasts, transfected with neurotrophin genes to provide a sustained supply of factors, and therefore provide a source of such factors for sustained periods. The use of such cells, or fibroblasts, to deliver the neurotrophins will enable regenerating axons to reinnervate their original targets and re-establish lost function, since the retinal ganglion cells continue to express neurotrophin receptors.

Previously, it had not been known if axons in chronic lesions of the CNS would respond to neurotrophins by regrowth. This is clearly an important clinical question because, there are a large number of patients with long standing CNS injuries who would benefit if such a treatment was found to be effective.

In a specific embodiment, the present invention thus achieves reinnervation of optic nerve targets in the superior colliculus and lateral geniculate body by grafting fibroblasts transfected with neurotrophin genes into the vitreous body of the eye. Such reinnervation has been achieved by the use of combinations of NGF, BDNF, NT3, FGF1 and FGF2 to promote the regeneration of retinal ganglion cells in the transected optic nerve when delivered to the vitreous body, particularly in combination, presumably because retinal ganglion cells express trk B and trk C after optic nerve injury. Since FGF1 and FGF2 also promote the survival of retinal ganglion cells after optic nerve section, FGFR1 and FGFR2 are probably also expressed, but the role of FGFs is likely to be complex, maintaining the viability of both grafted cells and host retinal ganglion cells and possibly also modulating the neurotrophin receptor affinity of the latter. The profile of expression of FGF1, FGF2, NGF, BDNF and NT3 mRNA and protein in both retinal ganglion cells and retinal glia in the acute period after optic nerve section and in long standing lesions, together with that of FGFR1, FGFR2, LNGFR, trk A, trk B, and trk C can advantageously affect the damaged or diseased CNS. Most surprisingly, sustained regeneration of retinal ganglion cell axons can be achieved by the intravitreal implantation of a combination of FGF2 + BDNF + NT3-transfected fibroblasts, correlating the response with FGFR1, FGFR2, trk B and trk C expression. In a further optional embodiment, NGF can be supplied to further enhance the regenerative process in the situation where LNGFR and trk A are expressed by retinal ganglion cells after optic nerve section.

Thus, the present invention contemplates the implantation of neurotrophin-transfected cells, and particularly fibroblasts, into the damaged or diseased portion of the central nervous system with or without nerve scar resection to effect regeneration. Resecting the old glial/collagen scar may be necessary in most chronic CNS lesions, since the glia limitans of the scar is believed to act as an impenetrable barrier to regenenerating axons. In such cases the cut ends of the nerve can be re-approximated, or the gap filled by implantation of bridging tissue. The properties of such tissue must include the promotion of axonal growth across the gap from proximal to distal stump, and co-operative interaction with CNS tissues beyond the lesion to permit re-entry of regenerating axons into the distal optic nerve stump. The present invention can utilize the properties of olfactory nerve glia (ensheathing cells) *in vitro,* since *in vivo* they permit axon growth throughout life along both the PNS and CNS trajectory of the olfactory pathway (Raisman, 1985; Doucette, 1990). Olfactory glia have similarities to, and differences from astrocytes and Schwann cells (Doucette, 1991). Adult ensheathing cells in culture provide a more effective substrate for regrowth of retinal ganglion cell neurites than either neonatal astrocytes or Schwann cells. The implantation of ensheathing cells between the cut ends of the adult optic nerve provides a method of integrating with astrocyte processes growing from both proximal and distal stumps. Moreover, axons invade the grafts from the proximal stump but fail to penetrate the distal optic nerve segment. Thus, unlike Schwann cells, ensheathing cells can facilitate the transfer of regenerating retinal ganglion cell axons into the segment of the nerve distal to the lesion site if growth is stimulated by neurotrophins.

Such methodology is of particular relevance to the development of effective strategies for the treatment of debilitation caused by the malformation of or injury to neural tissues of the CNS, and it is toward such objectives that the present invention is directed.

The neurotrophins of the present invention are notable in their ability to promote such neural growth in an environment that has been traditionally viewed as inhibitory to the growth promoting stimulus of known neurite outgrowth factors. Specifically, this inhibitory environment includes inhibitory molecular cues which are present on glial cells and myelin in the central nervous system.

The neurotrophins of the present invention are broadly selected from a group of neurotrophic factors, and more preferably the neurotrophins, ciliary neurotrophic factor (CNTF), neuron regulatory factor (NRF), acidic and basic fibroblast growth factor (FGF1 and FGF2), nerve growth factor (NGF), brain-derived neurotrophic factor (BDNF), and neurotrophin-3 (NT3). A highly preferred combination is that of FGF2, BDNF and NT3. These factors have been isolated, purified and characterized extensively in the prior art. See, for instance, U.S. Patent 5,180,820 which discloses BDNF, U.S. Patent 5,057,494 which discloses the use of both acid and basic FGF, and WO 95/006662, the disclosures of which are incorporated herein by reference.

The utilizable neurotrophic factors of the present invention also include fragments thereof and cognate molecules, congeners and mimics thereof which can be used to promote neurite growth in the CNS. In particular, the neurotrophins include molecules which contain structural motifs characteristic of these neurotrophic factors. Preferably, these structural motifs include those structurally similar to FGF2, BDNF or NT-3.

The invention extends to methods of promoting and enhancing neural regeneration *in vivo*, and to the corresponding genetic constructs, such as plasmids, vectors, transgenes, transfected cells, and the like, and to pharmaceutical compositions, all of which may be used to accomplish the objectives of such methods. More specifically, the neurotrophins of the present invention may be prepared as vectors or plasmids, and introduced into neural cells located at a site in the CNS where regeneration is needed, for example, by gene therapy techniques, to cause the expression of the combination of the neurotrophins of the present invention and to thereby promote the requisite neural growth. Gage *et al*., U. S. Patent 5,082670, issued January 21, 1992, whose disclosure is incorporated herein by reference, details various methods of delivery utilizable for the administration of the combinations of the present invention.

Another strategy contemplates the transfection of cells such as fibroblasts by the introduction of two or more of the neurotrophins or like agents contemplated by the present invention, followed by the introduction of such cells to the site of a lesion or other trauma, for the purpose of initiating regeneration. Similarly, the present method includes the formulation of two or more of the appropriate neurotrophins in a composition that may likewise be directly delivered to a CNS site, as by parenteral administration. In this mode, the neurotrophins of the combination of the present invention can thus be administered to a patient either in separate or combined dosage forms, admixed with a suitable pharmaceutically acceptable carrier. The carrier may be selected from a wide variety of forms depending the form of administration desired for administration, *e*.*g*., sublingual, rectal, nasal, intraventricular, intracerebral, oral or parenteral. If the compositions are to be injected directly into the patient's spinal cord, the carrier may be, for instance, an artificial cerebrospinal fluid. Controlled release formulations may also be used.

For pharmaceutical compositions to be administered parenterally, the carrier will usually comprise sterile water, although other ingredients to aid solubility, buffering or for preservation purposes may be included. Also, extenders may be added to compositions which are to be lyophilized. Injectable suspensions may also be prepared, in which case appropriate liquid carriers, suspending agents and the like may be employed. Examples of parenteral routes of administration are intravenous, intraperitoneal, intramuscular or subcutaneous injection. For the central nervous system, direct injection into the CNS is preferred, such as by intracerebral or interventricular injection or by injection into the cerebrospinal fluid or spinal cord. For such injection, catheters, needles and syringes may be used. Infusion of the neurotrophin combination via a catheter into the brain is an alternative method of administration.

A preferred example of a dosing regimen for the combinations of the present invention involves a bolus injection of the combination, followed by continuous infusion. Also, a sustained release dose or repeated delivery system of the combination may be used. A further alternative is a solid matrix containing the appropriate dosages of the combination which is implanted into the damaged region of the central nervous system.

Dosages of the combination of the present invention will depend upon the type of damage or disease under treatment, and the age, size and condition of the patient.

The present invention also includes transgenic mammals, especially mouse lines expressing a combination of two or more of the neurotrophins, and cells and tissues derived therefrom. in particular, the neurotrophins are BDNF, FGF2 and NT3.

More particularly, the present invention relates to the use of compositions delivering certain neurotrophins identified herein as "CNS neural growth modulators" (CNGMs), and particularly to a combination of neurotrophic factors as defined herein, to promote neurite outgrowth in the central nervous system (CNS). In general, neurons in the adult central nervous system have been considered incapable of regrowth, due to inhibitory molecular cues present on glial cells. The neurotrophins and methods of the present invention can be used to overcome such inhibition and promote CNS neurite outgrowth and regeneration.

The neurotrophins of the invention include and may be selected from any neurotrophic factor which is capable of modulating or promoting CNS neurite outgrowth, and particularly to nerve growth factor (NGF), acidic fibroblast growth factor (FGF-1), basic fibroblast growth factor (FGF-2), neurotrophin-3 (NT-3), brain derived neurotrophic factor (BNDF), agonists thereof, active fragments thereof, cognates thereof, congeners thereof, mimics, analogs, secreting cells and soluble molecules thereof. The invention also contemplates fragments of these molecules, and analogs, cognates, congeners and mimics of these molecules which have neurite-promoting activity.

The present invention relates in one aspect to the expression of combinations of these CNS neural growth modulators (CNGMs) or neurotrophic factors by fibroblasts *in vivo.* These molecules have been found to enhance neurite outgrowth and regeneration *in vivo,* in optic nerve crush experiments in transgenic animals. The increased neurite outgrowth-promoting capacity is proportional to the level of the combination of the neurotrophins expressed, and their period of expression. This is demonstrated by comparisons of combinations of the distinct transgenic fibroblasts of the invention, which express different neurotrophins, and by correlations following increased CNGM expression after a lesion of the optic nerve.

It should be appreciated that although optic nerves, both lesioned and unlesioned, are suitable for use with the present invention, that any part of the nervous system can likewise be used, including portions of the brain and spinal cord.

In a preferred embodiment, the combination of neurotrophins is BDNF, NT3 and FGF2, although combinations of at least two neurotrophic factors can also be utilized. The addition of NGF as a fourth component may also be advantageous in certain situations.

The present invention demonstrates that the inhibitory action of astroglial and oligodendroglial cells may be overcome, at least in part, by the neurite outgrowth promoting properties of the neurotrophins defined herein, and as particularly illustrated by the activity of a combination of BGNF, FGF2 and NT3 which allow enhancement of the regenerative capacity of the adult mammalian central nervous system following injury or disease.

As indicated earlier, the present invention extends to the promotion of neural growth in the CNS, including such growth as is desired to regenerate structures lost due to injury or illness, as well as those structures and tissues exhibiting incomplete or immature formation. The neurotrophins of the invention also exhibit a neuroprotective or neuropreservative effect as illustrated later on herein, and for example, could be administered to inhibit or counteract neural degeneration or loss independent of etiology.

The invention accordingly extends to constructs and compositions containing or delivering the neurotrophic factors of present invention, whether by the promotion of the expression of certain neurotrophins via gene therapy or the like, or by the exogenous administration of the neurotrophins where appropriate and beneficial, in pharmaceutical compositions to treat injured or diseased CNS structures. In this latter connection, it is contemplated that certain of the neurotrophins are able to exert a growth promoting effect when so administered, although it is recognized that members of the presently identified group, may prove more beneficial when delivered by means of expression. The invention is intended to extend to both routes and protocols where feasible.

It should also be appreciated that the present invention relates to the use of neurotrophin-secreting cells for the modulation of neural outgrowth, regeneration, and neural survival in the CNS. As such, certain soluble neurotrophins and fragments thereof, and cognate molecules thereof are also within the invention.

Therefore, if appearing herein, the following terms shall have the definitions set out below.

The terms "neurotrophins", "neurotrophic factors", "CNGM", "agents" and any variants not specifically listed, may be used herein interchangeably, and as used throughout the present application and claims refer to proteinaceous material including single or multiple proteins, and extends to those proteins having the amino acid sequence previously described and the profile of activities set forth herein and in the Claims. The foregoing terms also include active fragments of such proteins, cognates, congeners, mimics and analogs, including small molecules that behave similarly to said neurotrophins.

Accordingly, proteins displaying substantially equivalent or altered activity are likewise contemplated. These modifications may be deliberate, for example, such as modifications obtained through site-directed mutagenesis, or may be accidental, such as those obtained through mutations in hosts that are producers of the complex or its named subunits. Also, the terms "neurotrophins", and "neurotrophic factors" are intended to include within their scope proteins specifically recited herein as well as all substantially homologous analogs and allelic variations.

The amino acid residues described herein are preferred to be in the "L" isomeric form. However, residues in the "D" isomeric form can be substituted for any L-amino acid residue, as long as the desired functional property of immunoglobulin-binding is retained by the polypeptide. NH₂ refers to the free amino group present at the amino terminus of a polypeptide. COOH refers to the free carboxy group present at the carboxy terminus of a polypeptide. In keeping with standard polypeptide nomenclature, *J. Biol. Chem.,* **243**:3552-59 (1969), abbreviations for amino acid residues are shown in the following Table of Correspondence:

**TABLE OF CORRESPONDENCE**

| SYMBOL | AMINO ACID | |
|---|---|---|
| 1-Letter | 3-Letter | |
| Y | Tyr | tyrosine |
| G | Gly | glycine |
| F | Phe | phenylalanine |
| M | Met | methionine |
| A | Ala | alanine |
| S | Ser | serine |
| I | lie | isoleucine |
| L | Leu | leucine |
| T | Thr | threonine |
| V | Val | valine |
| P | Pro | proline |
| K | Lys | lysine |
| H | His | histidine |
| Q | Gln | glutamine |
| E | Glu | glutamic acid |
| W | Trp | tryptophan |
| R | Arg | arginine |
| D | Asp | aspartic acid |
| N | Asn | asparagine |
| C | Cys | cysteine |

It should be noted that all amino-acid residue sequences are represented herein by formulae whose left and right orientation is in the conventional direction of amino-terminus to carboxy-terminus. Furthermore, it should be noted that a dash at the beginning or end of an amino acid residue sequence indicates a peptide bond to a further sequence of one or more amino-acid residues. The above Table is presented to correlate the three-letter and one-letter notations which may appear alternately herein.

A "replicon" is any genetic element (e.g., plasmid, chromosome, virus) that functions as an autonomous unit of DNA replication *in vivo*; i.e., capable of replication under its own control.

A "vector" is a replicon, such as a plasmid, phage or cosmid, to which another DNA segment may be attached so as to bring about the replication of the attached segment.

A "DNA molecule" refers to the polymeric form of deoxyribonucleotides (adenine, guanine, thymine, or cytosine) in its either single stranded form, or a double-stranded helix. This term refers only to the primary and secondary structure of the molecule, and does not limit it to any particular tertiary forms. Thus, this term includes double-stranded DNA found, *inter alia,* in linear DNA molecules (e.g., restriction fragments), viruses, plasmids, and chromosomes. In discussing the structure of particular double-stranded DNA molecules, sequences may be described herein according to the normal convention of giving only the sequence in the 5' to 3' direction along the nontranscribed strand of DNA (i.e., the strand having a sequence homologous to the mRNA).

An "origin of replication" refers to those DNA sequences that participate in DNA synthesis.

A DNA "coding sequence" is a double-stranded DNA sequence which is transcribed and translated into a polypeptide *in vivo* when placed under the control of appropriate regulatory sequences. The boundaries of the coding sequence are determined by a start codon at the 5' (amino) terminus and a translation stop codon at the 3' (carboxyl) terminus. A coding sequence can include, but is not limited to, prokaryotic sequences, cDNA from eukaryotic mRNA, genomic DNA sequences from eukaryotic (e.g., mammalian) DNA, and even synthetic DNA sequences. A polyadenylation signal and transcription termination sequence will usually be located 3' to the coding sequence.

Transcriptional and translational control sequences are DNA regulatory sequences, such as promoters, enhancers, polyadenylation signals, terminators, and the like, that provide for the expression of a coding sequence in a host cell.

A "promoter sequence" is a DNA regulatory region capable of binding RNA polymerase in a cell and initiating transcription of a downstream (3' direction) coding sequence. For purposes of defining the present invention, the promoter sequence is bounded at its 3' terminus by the transcription initiation site and extends upstream (5' direction) to include the minimum number of bases or elements necessary to initiate transcription at levels detectable above background. Within the promoter sequence will be found a transcription initiation site (conveniently defined by mapping with nuclease S1), as well as protein binding domains (consensus sequences) responsible for the binding of RNA polymerase. Eukaryotic promoters will often, but not always, contain "TATA" boxes and "CAT" boxes. Prokaryotic promoters contain Shine-Dalgarno sequences in addition to the -10 and -35 consensus sequences.

An "expression control sequence" is a DNA sequence that controls and regulates the transcription and translation of another DNA sequence. A coding sequence is "under the control" of transcriptional and translational control sequences in a cell when RNA polymerase transcribes the coding sequence into mRNA, which is then translated into the protein encoded by the coding sequence.

A "signal sequence" can be included before the coding sequence. This sequence encodes a signal peptide, N-terminal to the polypeptide, that communicates to the host cell to direct the polypeptide to the cell surface or secrete the polypeptide into the media, and this signal peptide is clipped off by the host cell before the protein leaves the cell. Signal sequences can be found associated with a variety of proteins native to prokaryotes and eukaryotes.

The term "oligonucleotide", as used herein in referring to probes, is defined as a molecule comprised of two or more ribonucleotides, preferably more than three. Its exact size will depend upon many factors which, in turn, depend upon the ultimate function and use of the oligonucleotide.

The term "primer" as used herein refers to an oligonucleotide, whether occurring naturally as in a purified restriction digest or produced synthetically, which is capable of acting as a point of initiation of synthesis when placed under conditions in which synthesis of a primer extension product, which is complementary to a nucleic acid strand, is induced, i.e., in the presence of nucleotides and an inducing agent such as a DNA polymerase and at a suitable temperature and pH. The primer may be either single-stranded or double-stranded and must be sufficiently long to prime the synthesis of the desired extension product in the presence of the inducing agent. The exact length of the primer will depend upon many factors, including temperature, source of primer and use of the method. For example, for diagnostic applications, depending on the complexity of the target sequence, the oligonucleotide primer typically contains 15-25 or more nucleotides, although it may contain fewer nucleotides.

The primers herein are selected to be "substantially" complementary to different strands of a particular target DNA sequence. This means that the primers must be sufficiently complementary to hybridize with their respective strands. Therefore, the primer sequence need not reflect the exact sequence of the template. For example, a non-complementary nucleotide fragment may be attached to the 5' end of the primer, with the remainder of the primer sequence being complementary to the strand. Alternatively, non-complementary bases or longer sequences can be interspersed into the primer, provided that the primer sequence has sufficient complementarity with the sequence of the strand to hybridize therewith and thereby form the template for the synthesis of the extension product.

As used herein, the terms "restriction endonucleases" and "restriction enzymes" refer to bacterial enzymes, each of which cut double-stranded DNA at or near a specific nucleotide sequence.

A cell has been "transformed" by exogenous or heterologous DNA when such DNA has been introduced inside the cell. The transforming DNA may or may not be integrated (covalently linked) into chromosomal DNA making up the genome of the cell. In prokaryotes, yeast, and mammalian cells for example, the transforming DNA may be maintained on an episomal element such as a plasmid. With respect to eukaryotic cells, a stably transformed cell is one in which the transforming DNA has become integrated into a chromosome so that it is inherited by daughter cells through chromosome replication. This stability is demonstrated by the ability of the eukaryotic cell to establish cell lines or clones comprised of a population of daughter cells containing the transforming DNA. A "clone" is a population of cells derived from a single cell or common ancestor by mitosis. A "cell line" is a clone of a primary cell that is capable of stable growth *in vitro* for many generations.

Two DNA sequences are "substantially homologous" when at least about 75% (preferably at least about 80%, and most preferably at least about 90 or 95%) of the nucleotides match over the defined length of the DNA sequences. Sequences that are substantially homologous can be identified by comparing the sequences using standard software available in sequence data banks, or in a Southern hybridization experiment under, for example, stringent conditions as defined for that particular system. Defining appropriate hybridization conditions is within the skill of the art. See, e.g., Maniatis et al., *supra*; DNA Cloning, Vols. I & II, *supra;* Nucleic Acid Hybridization, *supra.*

A "heterologous" region of the DNA construct is an identifiable segment of DNA within a larger DNA molecule that is not found in association with the larger molecule in nature. Thus, when the heterologous region encodes a mammalian gene, the gene will usually be flanked by DNA that does not flank the mammalian genomic DNA in the genome of the source organism. Another example of a heterologous coding sequence is a construct where the coding sequence itself is not found in nature (e.g., a cDNA where the genomic coding sequence contains introns, or synthetic sequences having codons different than the native gene). Allelic variations or naturally-occurring mutational events do not give rise to a heterologous region of DNA as defined herein.

The phrase "pharmaceutically acceptable" refers to molecular entities and compositions that are physiologically tolerable and do not typically produce an allergic or similar untoward reaction, such as gastric upset, dizziness and the like, when administered to a human.

The phrase "therapeutically effective amount" is used herein to mean an amount sufficient to prevent, and preferably reduce by at least about 30 percent, more preferably by at least 50 percent, most preferably by at least 90 percent, a clinically significant change in the neurite growth promoting activity.

A DNA sequence is "operatively linked" to an expression control sequence when the expression control sequence controls and regulates the transcription and translation of that DNA sequence. The term "operatively linked" includes having an appropriate start signal (e.g., ATG) in front of the DNA sequence to be expressed and maintaining the correct reading frame to permit expression of the DNA sequence under the control of the expression control sequence and production of the desired product encoded by the DNA sequence. If a gene that one desires to insert into a recombinant DNA molecule does not contain an appropriate start signal, such a start signal can be inserted in front of the gene.

The term "standard hybridization conditions" refers to salt and temperature conditions substantially equivalent to 5x SSC and 65°C for both hybridization and wash.

In one aspect, the present invention relates to transgenic animals which express a combination of neurotrophins, in particular BGNF, FGF2 and NT3, and preferably in fibroblasts.

In a further embodiment, the present invention relates to certain therapeutic methods which would be based upon the activity of the combination of neurotrophins, their subunits, or active fragments thereof, or upon other drugs determined to possess the same activity. A first therapeutic method is associated with the promotion of CNS neural growth resulting from the presence and activity of the combination of the neurotrophins, their active fragments, analogs, cognates, congeners or mimics, and comprises administering a combination of such neurotrophins capable of promoting CNS development, regrowth or rehabilitation in the host.

The following examples are presented in order to more fully illustrate the preferred embodiments of the invention. They should in no way be construed, however, as limiting the broad scope of the invention.

### EXAMPLE 1

To promote regeneration of retinal ganglion cell axons, through acute and chronic lesions of the optic nerve into the lateral geniculate nucleus and superior colliculus, neurotrophic factors are applied directly to retinal ganglion cell somata by intravitreal transplantation of fibroblasts transfected with BDNF, NT3, NGF, FGF1 and FGF2.

The electronmicroscopy for these studies examines the fine details of axon/glia relationships in (I) the lesion, (ii) ensheathing glial cell bridges, (iii) distal stump and (iv) the reinnervation of the superior colliculus and lateral geniculate body. These studies use the technique of anterograde filling of retinal ganglion cell axons after injection of HRP into the vitreous body of the eye and employing DAB development.

### 1. Experimental models and reagents

(a) Optic nerve injury model The optic nerve is an ideal model for studying regeneration in the CNS. It is almost exclusively a unidirectional CNS tract of axons originating from retinal ganglion cells and projecting centrally mainly to the superior colliculus and lateral geniculate body. The nerve can be completely and unequivocally transected under direct vision, and regeneration monitored using the anti-GAP43 antibody which is a specific marker for regenerating fibers in the optic nerve since intact, unlesioned optic fibers do not contain this protein. Regenerating fibers are also detected by anterograde axon tracing studies after injecting rhodamine-B thiocyanate (Rh-B) into the vitreous body. Intravitreal injection of HRP will also unequivocally identify regenerated retinal ganglion cell axon terminals in central projection targets using DAB development and electronmicroscopy. The latter work is an essential prerequisite for providing the structural basis for studies on functional restitution, which can be accurately measured in the visual system by recovery of, for example, electrophysiological, light reflex and visually guided behavioural parameters. A quantitative estimate of the regenerative response in the nerve is also obtained by applying HRP distal to the lesion and counting the number of retrogradely filled retinal ganglion cells in retinal whole mounts developed by the TMB method. Retinal whole mounts also provide an ideal preparation for studying the changes in neurotrophin receptor, mRNA and protein expression after injury using standard *in situ* hybridization and immunocytochemical methods.
(b) Culture of neurotrophin- and FGF-2-expressing cells Cell lines derived from cultures of normal, Fischer 344, rat dermal fibroblasts transfected with DNA encoding NGF, BDNF, FGF-2 (both the secreted form and a form which remains cell-associated as a high molecular weight complex) and NT-3 were obtained through collaboration with the Gage laboratory at the Salk Institute, San Diego. These cell lines also coexpress the gene conferring gentamicin resistance which, in the initial isolation of lines, is selected for by including the drug G418 in the medium. Expression of both transgenes is maintained by continued inclusion of the drug in culture media. The parental fibroblast line used for DNA transfection is also available as a control population of cells. The designations of the cell lines we have used in pilot studies are thus as follows: FF12 (parental, untransfected dermal fibroblasts);
   FF12/FGFppN (expressing secreted FGF-2); FF12/B-11 (expressing cell-associated FGF-2); FF12/BN1 (expressing BDNF); FF12/NGF (expressing NGF), and FF12/NT3 (expressing NT-3). Cell stocks are grown in Dulbeccos Modified Eagles Medium plus 10 (fetal calf serum (DMEM/F10) including G418 at 400 g/ml for all but the parental cell line. Cells grow with characteristics of normal fibroblasts, show contact-inhibition of growth and poor growth at low density. They do not form tumors after implantation and thus have advantages for implantation studies over the more widely-used transformed or tumour-derived cell lines which are tumorigenic *in vivo*.
(c) Preparation of cells for implantation The method developed for the implantation of myoblasts into dystrophic muscles (Partridge T. and Beauchamp J, personal communication) was used. Cells of either a single ceil line, or cell lines in combination (5.10⁶), can be pelleted by centrifugation in small Eppendorf tubes and incorporated into a fibrin clot by resuspending in a solution of the clottable protein, Tisseel (Immuno Ltd.), with or without aprotinin to retard clot resorption *in vivo,* and thrombin to effect clot formation by precipitation of insoluble protein. Solid gel-like clots containing known numbers of tightly-packed cells are implanted in the vitreous. In the absence of aprotinin, clot resorption and outward cell migration occurs more rapidly than in the presence of the protease inhibitor.
(d) Immunocytochemistry and molecular biology Both the ensheathing cell bridged and unbridged lesion site in the optic nerve are analyzed qualitatively and quantitatively by immunohistochemistry. The antibodies used to detect and identify cellular elements within lesions are commercially available and include rabbit anti-bovine glial fibrillary acidic protein (GFAP) for astrocytes and ensheathing cells; monoclonal 192 against p75 LNGFR for ensheathing cells; rabbit anti-carbonic anhydrase II (CAII) for oligodendrocytes; rabbit antibodies to antigens ED1 and OX47 for monocytes, macrophages and microglia; and rabbit anti-mouse sarcoma laminin, rabbit anti-fibronectin, rabbit anti-collagen I, II and IV and monoclonal anti-chondroitin-6-sulphate proteoglycan, for matrix molecules. Axonal markers include GAP43, RT97, peripherin and iii tubulin. Image analysis of the lesion site allows the extent of antigen-specific immunofluorescence to be quantified by measuring the relative intensity in longitudinal sections of the nerve taken from the mid-sagittal plane (Logan et al.,1994b; Logan and Berry, 1994). The expression of FGF1, FGF2, BDNF, NT3, NGF, and their receptors are documented in the retinal whole mounts from optic nerve lesioned rats. Previously used probes and antibodies, including northern blotting, ribonuclease protection assay, *in situ* hybridization, ELISA, western blotting, and immunohistochemistry are utilized to quantitate the expression (for methods see Logan et al., 1992a,b; Logan et al., 1994a,b).
(e) Primary culture of ensheathing cells Primary cultures of olfactory nerve glia are prepared from carefully dissected tissues from adult (> 3 months-old) F344 rats of either sex, as follows. The nerve rootlets are dissected from the intracranial surface of the cribriform plate of the ethmoid bone without disturbing the adjacent anterior ethmoid branch of the nasociliary nerve. Sheets of rootlets are peeled away from the olfactory bulbs where they comprise the nerve layer, having first stripped the bulb of pia-arachnoid. Cell suspensions obtained from trypsin digests of olfactory nerve tissue are then inoculated into culture flasks or onto cover slips precoated with polylysine (PLL) and then laminin (Sigma), each at 10 (g/ml. Cells are grown in DMEM (Imperial), supplemented with 10( batch-tested fetal calf serum (Globepharm and PAA) (Sonigra et al., in press). At confluence, cells are harvested and selected by immunoadsorbtion (panning) for expression of p75 LNGFR, using the monoclonal 192 antibody. Selected cells are expanded for several days before implanting in Tisseel (see 1(c) above).
(f) Optic nerve lesions The optic nerve is lesioned in the orbit according to the method of Berry et al. (1988a,b), and neurotrophin-transfected fibroblasts implanted into the retina at the same time (see Berry et al., (1996) for methods).

### 2. Experimental design

Fischer 344 adult rats (250-300g) of either sex will be used throughout since they are consanguineous with both the transfected fibroblast and the ensheathing glial cells we will use. Groups of 5 and 10 animals are used for qualitative and quantitative analysis respectively.

(i) Expression of neurotrophins and their receptors in retinal ganglion cells after optic nerve lesions. (a) After transection of the optic nerve, retinal whole mounts are prepared from groups of animals at 2, 5, 10, 20, 40 and 80 days post lesion (dpl). Protein and mRNA for FGF1, FGF2, BDNF, NT3 and NGF and their receptors is analyzed immunocytochemically and by *in situ* hybridization in retinal whole mounts to resolve the cellular localization of these products. Protein and mRNA for the above neurotrophins is extracted from freshly isolated retina in groups animals at the same ages post-lesion and detected quantitatively by ribonuclease protection assay, northern and western blotting. (b) The same procedures as in (a) above are repeated in a separate group of animals after optic nerve section and vitreal implantation of a combination of FGF2 + BDNF + NTS (+NGF, if LNGFR and /or trk A are detected in (ia) above). (c) The same procedures as in (a) above are repeated in another group of animals in which neurotrophin-transfected cells are implanted at 80 dpl into the vitreous body of the eye, in the same combination as (b) above, with and without optic nerve scar resection, and with and without ensheathing cell bridging implants. These latter experiments provide data about the neurotrophin status of the retina 80 dpl, and how it may alter after retinal implantation of transfected cells, optic nerve relesion, and implantation of ensheathing cells into the old scar site. Controls for these experiments are optic nerve lesioned animals either without vitreal grafts or with vitreal implants of the untransfected parental fibroblast cell line. Adult unlesioned retina acts as an additional control for base-line levels. Bridging implants of perineural fibroblasts acts as a additional control.

(ii) Regeneration of retinal ganglion cell axons to the superior colliculus and lateral geniculate body after acute optic nerve lesions. The optic nerve is transected and a combination of FGF2 + BDNF + NT3 (+ NGF)-transfected fibroblasts implanted into the eye. At 40 and 80 dpl, groups of animals are killed to establish the pattern of regrowth of retinal ganglion cell axons into the superior colliculus and lateral geniculate body. (These times post-lesion are chosen since it is likely, as judged from our previous findings after intravitreal peripheral nerve transplantation, that at 40 dpl axons will be entering these targets and by 80 days reinnervation will be complete, providing there is sustained release of neurotrophins from the transfected implants and retinal ganglion cells continue to express the appropriate receptors). Regeneration is be detected using GAP43 immunocytochemistry and anterograde axon tracing on sections of a whole mount of the optic nerve, optic chiasma and optic tract; reinnervation of the superior colliculus and lateral geniculate body will be assessed in coronal sections through these nuclei. A quantitative measure of the number of axons reinnervating the superior colliculus will be obtained by applying HRP to the superior colliculus and preparing retinal whole mounts 48 hours later and counting the number of HRP filled retinal ganglion cells after development by the TMB method. The synaptology of reinnervated terminals is studied using the technique of anterograde HRP tracing and DAB development in conjunction with electronmicroscopy.

(iii) Properties of olfactory ensheathing cells as bridging tissue implanted between the cut ends of the optic nerve. This experiment is essentially a replication of (ii) above except that ensheathing cells will be implanted into the optic nerve lesion, and the growth of optic fibers through the bridged lesion assessed qualitatively and quantitatively and compared with the results obtained in (ii) above. The interface between the bridging grafted tissue and the distal and proximal optic nerve segments, together with axon-glia relationships within the grafts is studied immunocytologically using the qualitative and quantitative methods outlined in 1(d) above, and by electronmicroscopy. Control tissue for bridging the gap between distal and proximal stunts of the optic nerve will comprise perineural cells.

(iv) Regeneration of retinal ganglion cell axons to the superior colliculus and lateral geniculate body after chronic optic nerve lesions. (a) The optic nerve is transected and at 80 dpl a combination of FGF2 + BDNF + NT3 (+ NGF)-transfected cells implanted into the eye. Groups of animals are killed 40 and 80 days after implantation to establish the pattern of regrowth distally from the lesion into the superior colliculus and lateral geniculate body as in (ii) above. (b) A further group of animals is prepared as in (iiia) above and the scar resected at the same time as vitreal implantation of tranfected cells. This experimental group is subdivided into 2 further groups; in one of which the optic nerve stumps is anastomosed by suture; in the other the gap between the resected stumps is filled with a Tisseel clot containing olfactory nerve ensheathing glia. In both groups regeneneration is assessed as in (iia) and (iii) above.

### EXAMPLE 2

Experiments were conducted to determine if the supply of neurotrophins (NTs) is the primary determinant of retinal ganglion cell (RGC) regeneration in the severed optic nerve. The experiments comprised 9 groups of 8-12 adult Wistar rats in which the optic nerve was crushed intra-orbitally: one group had a sham-implant operation; 6 groups were all implanted with gel pellets containing fibroblasts transfected to express a) FGF-2, b) NT-3, c) BDNF, d) FGF-2 and NT-3, e) FGF-2 and BDNF or f) FGF-2, NT-3 and BDNF; two (2) further control groups included implants of untransfected fibroblasts (F12) or heat-killed transfected fibroblasts. The rats were left for 20 or 50 days and their optic nerves and retinae prepared for immunohistochemical examination of the cellular reaction to injury. Anterograde axon tracing with rhodamine-B provided unequivocal qualitative evidence of regeneration in each group. The number of HRP filled retinal ganglion cells (RGCs) (which can be counted in retinal whole mounts) after injection of HRP at a site 2mm distal to the lesion gives a direct measure of the numbers of axons growing across the lesion (see Table 1 below).

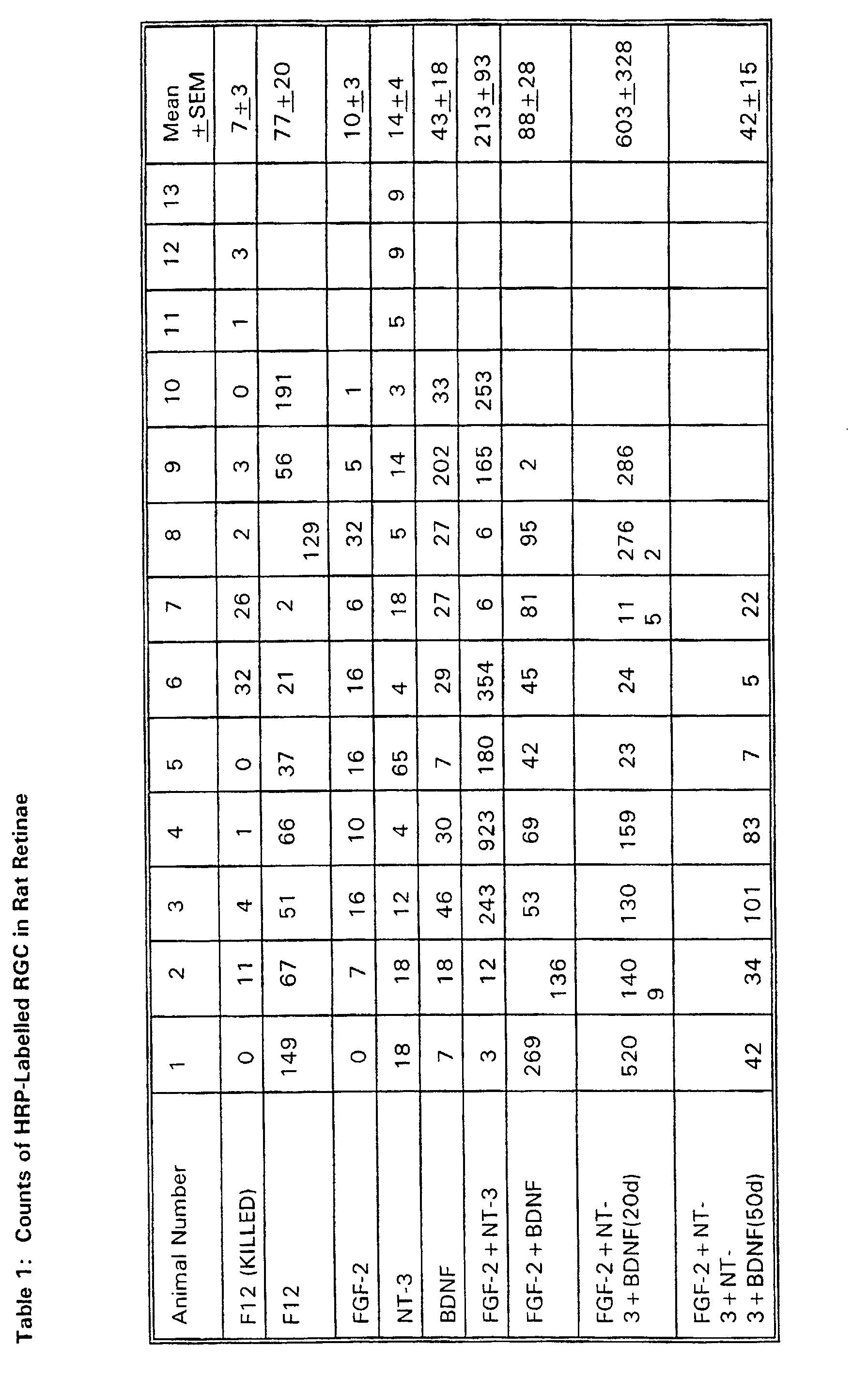

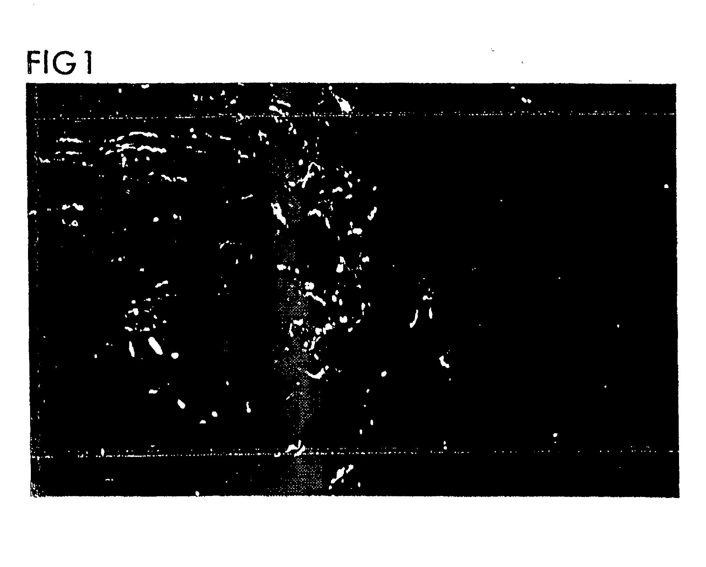

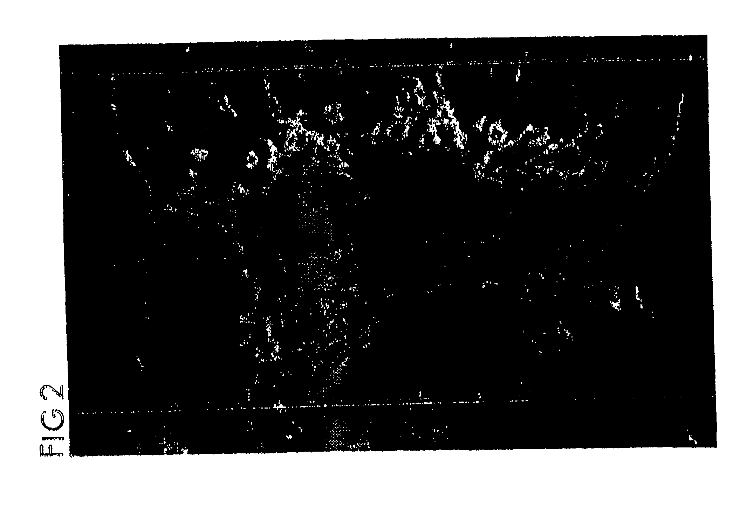

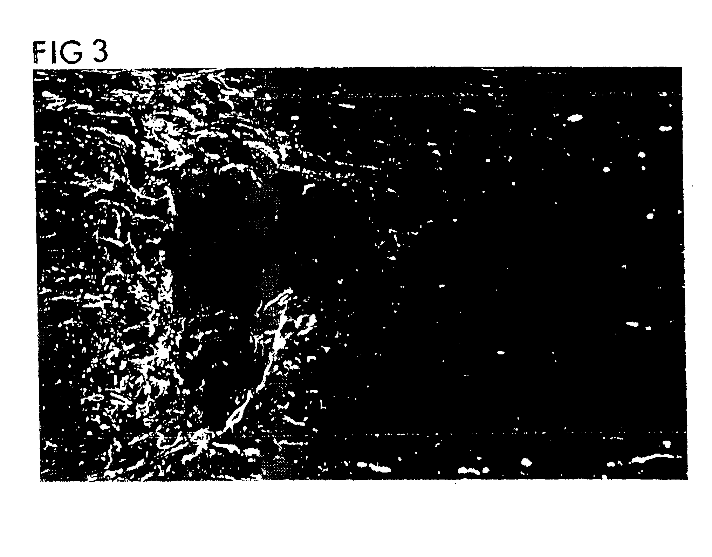

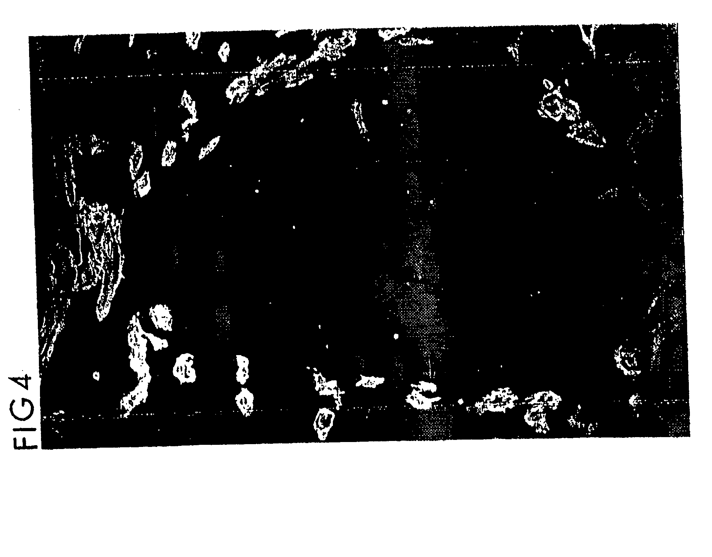

In the control groups most RGCs become degenerate in the retina (mean counts of HRP-positive RGC in the two groups = 0-191) and few if any GAP 43 (growth associated protein) positive axons were present in the proximal nerve segment (see FIGURE 1). No fibers crossed the lesion site and dense scar material was deposited in the wound (see FIGURE 2). In the single or double NT implants there was a small increase in the number of HRP-positive RGCs in the retinae (mean RGC counts in the 5 groups range from 10-213) and GAP 43 positive axons in the proximal segment of the nerve. Those animals receiving all three Nts showed the greatest number of HRP-positive RGCs (25-2762, mean = 603), with significant numbers of GAP 43 positive axons regenerating 3-5 mm into the distal nerve segment (see FIGURE 3). Furthermore, in this group a glia/mesenchymal scar was not formed at the wound site (see FIGURE 4). These results indicate that perineural delivery of specific NT combinations can mobilize and maintain axon regeneration for at least 20 days and that these regenerating fibers interfere with scar formation. The unsustained nature of the growth response may reflect discontinuity of neurotrophin delivery.

Thus, from these results, it may be concluded that:
Neurotrophin administration to the cell body enhances RGC survival and axon regeneration;
Specific combinations are more effective than single neurotrophins;
Regenerating axons suppress scar formation at the lesion site;
Long-term RGC survival and regeneration requires sustained supply of neurotrophins.

### EXAMPLE 3

Fibroblasts transfected with basic fibroblast growth factor (FGF2), brain derived neurotrophic factor (BDNF), and neurotrophin-3 (NT3) genes promote robust regeneration of axons in the crushed optic nerve, for substantial distances beyond the lesion site by 20 days after injury, by vitreal grafting. To ascertain if this regenerative response is sustained through the optic nerve, optic chiasma, and optic tract into the superior colliculus and lateral geniculate body retinal ganglion cells are exposed to combinations of neurotrophins secreted by implanted transfected fibroblasts. At the same time, the expression of neurotrophin receptors (LNGFR, trk A, trk B, trk C, FGFR1and FGFR2), and their MRNAS is monitored in order to correlate the findings with the regenerative response. It is also relevant to determine if the application of the above neurotrophic factors to retinal ganglion cells in rats with chronically lesioned optic nerves stimulates axon regrowth either through the old lesion, or through grafted bridging tissue, made up of olfactory nerve ensheathing glia, introduced between proximal and distal stumps of the optic nerve after resection of the scar. Any regenerative response will be correlated with neurotrophin receptor expression by retinal ganglion cells. The pattern of axon regrowth in both experiments is assessed by retrograde and anterograde axon tracing techniques both quantitatively and qualitatively, and axon/glia relationships in the lesion, within bridging grafts and throughout the course of distal trajectories monitored immunocytochemically and with the electron microscope. Synaptology within projection target nuclei is also studied by electronmicroscopy.

The following is a list of documents related to the above disclosure and particularly to the experimental procedures and discussions. The documents should be considered as incorporated by reference in their entirety.
Aguayo (1985) "Axonal regeneration from injured neurons in the adult mammalian central nervous system," In: Synaptic Plasticity (Cotman, C.W., ed.) New York, The Guilford Press, pp. 457-484.)
Allendoeffer, K. L. , Cabelli, R. J. , Escandon, E., Kaplan, D.R., Nikolics, K. & Shatz, C. J. (1994) Journal of Neuroscience **14**: 1795-811.
Baird, A. (1992) Current Opinion in Neurobiology **4**: 78-86.
Berry, M., Rees, L., Hall, P., Yui, P. & Sievers, J. (1988a) Brain Research Bulletin **20**: 223-31.
Berry, M., Hall, S., Follows, R., Rees, L., Gregson, N. & Sievers, J. (1988b)
Journal of Neurocytology **17**: 727-44.
Berry, M., Hall, S., Schewan, D. & Cohen, J. (1994) Eye **8**: 245-54.
Berry, M., Carlile, J. & Hunter, A. (1996) Journal of Neurocytology **25**:147-170.
Bugra, K., Oliver, L., Jacquemin, E., Laurent, M., Courtois, Y. & Hicks, D.(1993)
European Journal of Neuroscience **5**: 1586-95.
Carlstedt et al. (1989) *Brain Res. Bull.* **22**:93-102.
Carri, N.G., Richardson, P. & Ebendal, T. (1994) International Journal of Developmental Neuroscience **12**: 567-78.
Castillo, B., Delcerro, M., Breakfield, X. O., Frim, D. M., Barnstable, C. J., Dean, D. O. & Bohn, M. C. (1994) Brain Research **647**: 30-6.
Delarosa, E. J., Arribas, A., Frade, J. M. & Rodriguez-Tebar, A. (1994) Neuroscience **58**: 347-52.
Doucette, R. (1991) Journal of Comparative Neurology **312**: 451-66.
Doucette, R. (1990) Glia **3**: 433-49.
Elkabes, S., Schaar, G. G., Dreyfus, C. F. & Black, I. B. (1995) Neuroscience **66**: 879-89.
Escandon, E., Soppet, D., Rosenthal, A., Mendozaramirez, J. L., Szonyi, E., Burton, L. E., Henderson, C. E., Parada, L. F. & Nikolics, K. (1994) Journal of Neuroscience **14**: 2045-68.Fawcett et al. (1990) *Annu. Rev. Neurosci* **13**:43-60.
Hertzog, K. H., Bailey, K. & Burde, Y. A.(1994) Development **120**: 1643-9. Ikeda, T. & Puro, D. J. (1994) Brain Research **649**: 260-4.
Ishigooka, H., Kitaoka, T., Boutilier, S. B., Bost, L. M., Aotakikeen, A. E., Tablin, F. & Hjelmeland, L. M. (1993) Investigative Ophthalmology and Visual Science **34**: 2813-23.
Jelsma, T. N., Friedman, H. H., Berkelaar, M., Bray, G. M. & Aguayo, A. J. (1993) Journal of |Neurobiology **24**: 1207-14.
Johnson, A. R. (1993) Bioessays **15**: 807-13.
Kalderon (1988) *J. Neurosci Res.* **21**:501-512.
Keynes, R. J. & Cook, G. M. W. (1995) Current Opinion in Biology **5**: 75-82. Khan, S. & Wigley, C. B. (1994) Neuro Report **5**: 1381-5.
Kliot et al. "Induced regeneration of dorsal root fibers into the adult mammalian spinal cord," In: *Current Issues in Neural Regeneration,* New York, pp. 311-328; Koide, J., Takahashi, J. B., Hoshimaru, M., Kojima, M., Otsuka, T., Asahi, M.. & Kikushi, H. (1995) Neuroscience Letters **185**: 183-6.
Lillien, L. (1994) Perspectives on Developmental Neurobiology **2**: 175-82.
Lillien, L. & Cepko, C. (1992) Development **115**: 253-66.
Logan, A., Frautschy, S. A., Gonzalez, A. M. & Baird, A. (1992a) Journal of Neuroscience **12**:3828-37.
Logan, A., Frautschy, S. A., Gonzalez, A. M. & Baird, A. (1992b) Brain Research **587:** 216-25.
Logan, A. & Berry, M. (1994) *In:* Providing Pharmacological Access to the Brain. Alternative Approaches. (Ed. Flanagan, T. R., Emerich, D. F. & Winn, S. R.) Academic Press, San Diego. pp 3-19.
Logan, A., Oliver, J. J. & Berry, M. (1994a) Progress in Growth Factor Research **5**: 1-18.
Logan, A., Berry, M., Gonzalez, A. M., Frautschy, S. A., Sporn, M. B. & Baird, A. (1994b) European Journal of Neuroscience **6**: 355-33.
Malecaze, F., Mascarelli, F., Bugra, K., Fuhrmann, G., Courtois, Y. & Hicks, D. (1993) Journal of Cellular Physiology **154**: 631-42.
Mansourrobaey, S., Clarke, D. B., Wang, Y. C., Bray, G. M. & Aguayo, A. J. (1994) Proceedings of the National Acadamey of Science of USA **91**: 1632-6. Martini (1994) *J. Neurocytol.* **23**:1-28)
Mascarelli, F., Torriglia, A., Soubrane, G., Hichs, D., Malecaze, F. & Courtois, Y. (1991)Annales DEndocrinologie **52**: 441-6.
Mey, J. & Thanos, S. (1993) Brain Research **602**:304-17.
Miller et al. (1985) *Develop. Biol*. **111**:35-41
Monard, D. (1998) Trends in Neurosciences **11**: 541-4.
Perez, M. T. R. & Caminos, E. (1995) Neuroscience Letters **183**: 96-9.
Perry, J., Du, J., Kjeldbye, H. & Guras, P. (1995) Current Eye Research **14**: 237-54.
Pollerberg et al. (1985) *J. Cell. Biol*. **101**:1921-1929.
Ramon-Cuet, A. & Nieto-Sampedro, M. (1994) Experimental Neurology **127**: 232-44.
Richma, D. W. & Brecha, N. C. (1995) Visual Neuroscience **12**: 215-22.
Rodriguez-Tebar, A., Delarosa, E. J. & Arribas, A. (1993) European Journal Biochemistry **211**: 789-94.
Romanic, A. M. & Madri, J.A. (1994) Brain Pathology **4**: 145-56.
Silver et al. (1982) *J. Comp. Neurol*. **210**:10-29; ;
Sonigra, R. J., Kandiah, S. S. & Wigley, C. B. (1995) Glia; in press.
Stuermer et al. (1992) *J. Neurobiol*. **23**:537-550.
Takahashi, J. B., Hoshimaru, M., Kikushi, H. & Hatanaka, M. (1993) Neuroscience Letters **151**: 174-7.
Tcheng, M., Oliver, L., Courtois, Y. & Jeanny, J. C. (1994a) Experimental Cell Research **212**: 30-5.
Tcheng, M., Fuhrmann, G., Hartmann, M. P., Courtois, Y. & Jeanny, J. C. (1994b) Experimental Eye Research **58**: 351-8.
Torriglia, A. & Blanquet, P. R. (1994) Neuroscience **60**: 969-81.
Torriglia, A., Jeanny, J. C. & Blanquet, P. R. (1994) Neuroscience Letters **172**: 125-8.
Weibel, D., Kreutzberg, G. W. & Schwab, M. E. (1995) Brain Research **679**: 249-54.

While the invention has been described and illustrated herein by references to various specific material, procedures and examples, it is understood that the invention is not restricted to the particular material combinations of material, and procedures selected for that purpose. Numerous variations of such details can be implied as will be appreciated by those skilled in the art.

## Claims

1. The use of a combination of at least two neurotrophins capable of enhancing neurite outgrowth, or fragments thereof capable of enhancing neurite outgrowth, or analogs thereof capable of enhancing neurite outgrowth, or cells secreting neurotrophin molecules capable of enhancing neurite outgrowth in the manufacture of a medicament for the promotion of neural growth in vivo in diseased or injured tissues of the central nervous system of a mammal.

2. The use of Claim 1 wherein said neurotrophins are selected from the group consisting of nerve growth factor (NGF), acidic fibroblast growth factor (FGF-1), basic fibroblast growth factor (FGF-2), neurotrophin-3 (NT-3), brain-derived neurotrophic factor (BNDF).

3. The use of Claim 1 or 2 wherein said neurotrophins are deliverable to the central nervous system via secreting cells which express said factors.

4. The use of Claim 3 wherein said secreting cells are transfected fibroblasts expressing said factors.

5. The use of Claim 2 wherein said neurotrophins are basic fibroblast growth factor (FGF-2), neurotrophin-3 (NT-3) and BNDF.

6. The use of Claim 1 wherein the medicament is administerable via a perineural route.

7. The use of Claim 2 which additionally includes the use of nerve growth factor NGF.

8. A recombinant DNA molecule for use according to any of Claims 1-6, comprising two or more genes encoding neurotrophins, or fragments thereof capable of enhancing neurite outgrowth, or analogs thereof capable of enhancing neurite outgrowth, associated with an expression control sequence.

9. A vector comprising the recombinant DNA molecule of Claim 8.

10. A non-human transformed host containing the vector of Claim 9.

11. A non-human transgenic mammal comprising secreting cells which express two or more neurotrophins.

12. The transgenic mammal of Claim 11, wherein the secreting cells are fibroblasts.

13. The transgenic mammal of Claim 11, wherein the neurotrophins are brain-derived neurotrophic factor (BDNF), basic fibroblast growth factor (FGF2) and neurotrophin-3 (NT3).

14. A cell culture comprising the fibroblast cells of the transgenic mammal of Claim 13.

15. A cell culture system comprising tissue from the central nervous system of the transgenic mammal of Claim 11.

16. An in-vitro method for enhancing neuronal outgrowth of CNS neurons, comprising culturing said neurons on the cell culture system of Claim 14.

17. An in vitro method for enhancing neuronal outgrowth of CNS neurons, comprising culturing said neurons on the cell culture system of Claim 15.

## Patentansprüche

1. Verwendung einer Kombination von mindestens zwei Neurotrophinen, die zur Steigerung von Neuritauswuchs geeignet sind, oder Fragmenten davon, die zur Steigerung von Neuritauswuchs geeignet sind, oder Analoga davon, die zur Steigerung von Neuritauswuchs geeignet sind, oder Neurotrophinmoleküle sekretierende Zellen, die zur Steigerung von Neuritauswuchs geeignet sind, zur Herstellung eines Medikaments für die Förderung des Nervenwachstum *in vivo* in erkrankten oder geschädigten Geweben des zentralen Nervensystems eines Säugers.

2. Verwendung nach Anspruch 1, wobei die Neurotrophine aus der Gruppe, bestehend aus Nervenwachstumsfaktor (NGF), saurem Fibroblastenwachstumsfaktor (FGF-1), basischem Fibroblastenwachstumsfaktor (FGF-2), Neurotrophin-3 (NT-3), Him-abstammendem neurotrophen Faktor (BNDF), ausgewählt sind.

3. Verwendung nach Anspruch 1 oder 2, wobei die Neurotrophine durch sekretierende Zellen, welche diese Faktoren exprimieren, in das zentrale Nervensystem lieferbar sind.

4. Verwendung nach Anspruch 3, wobei die sekretierenden Zellen transfizierte Fibroblasten sind, welche die Faktoren exprimieren.

5. Verwendung nach Anspruch 2, wobei die Neurotrophine basischer Fibroblastenwachstumsfaktor (FGF-2), Neurotrophin-3 (NT-3) und BNDF sind.

6. Verwendung nach Anspruch 1, wobei das Medikament über einen perineuralen Weg verabreichbar ist.

7. Verwendung nach Anspruch 2, welche zusätzlich die Verwendung des Nervenwachstumsfaktors NGF beinhaltet.

8. Rekombinantes DNA-Molekül zur Verwendung nach einem der Ansprüche 1 bis 6, umfassend zwei oder mehr Gene, welche für Neurotrophine oder Fragmente davon, die zur Steigerung von Neuritauswuchs geeignet sind, oder Analoga davon, die zur Steigerung von Neuritauswuchs geeignet sind, kodieren, assoziiert mit einer Expressionskontrollsequenz.

9. Vektor, welcher das rekombinante DNA-Molekül nach Anspruch 8 umfaßt.

10. Nicht-menschlicher, transformierter Wirt, der den Vektor nach Anspruch 9 enthält.

11. Nicht-menschlicher, transgener Säuger, der sekretierende Zellen, welche zwei oder mehr Neurotrophine exprimieren, umfaßt.

12. Transgener Säuger nach Anspruch 11, wobei die sekretierenden Zellen Fibroblasten sind.

13. Transgener Säuger nach Anspruch 11, wobei die Neurotrophine Himabstammender neurotropher Faktor (BDNF), basischer Fibroblastenwachstumsfaktor (FGF2) und Neurotrophin-3 (NT3) sind.

14. Zellkultur, welche die Fibroblastenzellen des transgenen Säugers von Anspruch 13 umfaßt.

15. Zellkultursystem, welches Gewebe aus dem zentralen Nervensystem des transgenen Säugers von Anspruch 11 umfaßt.

16. *In vitro*-Verfahren zur Steigerung des Neuritauswuchs von ZNS-Neuronen, welches das Züchten der Neuronen auf dem Zellkultursystem nach Anspruch 14 umfaßt.

17. *In vitro*-Verfahren zur Steigerung des Neuritauswuchs von ZNS-Neuronen, welches das Züchten der Neuronen auf dem Zellkultursystem nach Anspruch 15 umfaßt.

## Revendications

1. Utilisation d'une combinaison d'au moins deux neurotrophines capables de favoriser l'excroissance de neurites ou de leurs fragments, capables de favoriser l'excroissance de neurites ou de leurs analogues, capables de favoriser l'excroissance de neurites ou de cellules sécrétant des molécules de neurotrophine, capables de favoriser l'excroissance de neurites dans la fabrication d'un médicament pour la promotion de la croissance neurale in vivo dans des tissus malades ou blessés du système nerveux central d'un mammifère.

2. Utilisation selon la revendication 1, dans laquelle lesdites neurotrophines sont choisies dans le groupe comprenant le facteur de croissance de nerfs (NGF), le facteur de croissance de fibroblastes acides (FGF-1), le facteur de croissance de fibroblastes basiques (FGF-2), le facteur neurotrophique (BNDF) dérivé du cerveau et le facteur de neurotrophine-3 (NT-3).

3. Utilisation selon la revendication 1 ou 2, dans laquelle lesdites neurotrophines sont aptes à être délivrées au système nerveux central par l'intermédiaire de cellules de sécrétion qui expriment lesdits facteurs.

4. Utilisation selon la revendication 3, dans laquelle lesdites cellules de sécrétion sont des fibroblastes transfectés exprimant lesdits facteurs.

5. Utilisation selon la revendication 2, dans laquelle lesdites neurotrophines sont le facteur de croissance de fibroblastes basiques (FGF-2), de neurotrophine-3 (NT-3) et de BNDF.

6. Utilisation selon la revendication 1, dans laquelle le médicament est apte à être administré par la voie périneurale.

7. Utilisation selon la revendication 2, qui comprend, en outre, l'utilisation du facteur de croissance de nerfs NGF.

8. Molécule d'ADN recombinante pour l'utilisation selon l'une quelconque des revendications 1 à 6, comprenant deux ou plusieurs neurotrophines codant des gènes ou leurs fragments, capables de favoriser l'excroissance de neurites ou leurs analogues, capables de favoriser l'excroissance de neurites, associées à une séquence de régulation d'expression.

9. Vecteur comprenant la molécule d'ADN recombinante selon la revendication 8.

10. Hôte transformé non humain renfermant le vecteur selon la revendication 9.

11. Mammifère transgénique non humain comprenant les cellules de sécrétion qui expriment deux ou plusieurs neurotrophines.

12. Mammifère transgénique selon la revendication 11, dans lequel les cellules de sécrétion sont des fibroblastes.

13. Mammifère transgénique selon la revendication 11, dans lequel les neurotrophines sont le facteur neurotrophique dérivé du cerveau (BDNF), le facteur de croissance de fibroblastes basiques (FGF2) et la neurotrophine-3 (NT3).

14. Culture de cellules comprenant les cellules de fibroblastes du mammifère transgénique selon la revendication 13.

15. Système de culture de cellules comprenant du tissu du système nerveux central du mammifère transgénique selon la revendication 11.

16. Procédé in vitro pour favoriser l'excroissance neuronale de neurones CNS, comprenant la culture desdits neurones sur le système de culture de cellules selon la revendication 14.

17. Procédé in vitro pour favoriser l'excroissance neuronale de neurones CNS, comprenant la culture desdits neurones sur le système de culture de cellules selon la revendication 15.
